# EUROPEAN PATENT APPLICATION

(11) **EP 0 740 941 A1**
(43) Date of publication of application: **06.11.1996**
(21) Application number: 96105564.7
(22) Date of filing: 09.04.1996
(51) Int. Cl.: A61L 9/12

(54) **Container for vaporizable solutions**

(30) Priority: 02.05.1995 IT BO950073
(71) Applicant: FALP S.r.l., I-40060 Passo Segni Baricella, Bologna (IT)
(72) Inventor: Falchieri, Roberto, 40060 Baricella (Bologna) (IT)
(74) Representative: Modiano, Guido, Dr.-Ing.

(57) **Abstract**

The container (1) for vaporizable solutions has a neck (3) through which a portion of a wick (2) protrudes. The container (1) has a sealing gasket (11) that is fitted by pressing onto the neck (3). The gasket (11) axially forms a tubular passage through which the wick (2) is fitted so that it passes hermetically; the gasket (11) is provided with a lip (15) along its peripheral region, and the lip (15) is folded downwards and adapted to engage hermetically the upper edge (10) of the neck (3). A closure (5) is screwed onto the neck (3) and is provided with an annular ridge (18) that is adapted to abut against the sealing gasket (11). The closure (5) axially forms a tubular portion (8) that is closed at the upper end and is adapted to accommodate, with a limited interspace, the portion of the wick (2) that protrudes from the neck (3) of the container (1).

## Description

The present invention relates to a container for insecticidal solutions and the like for use in devices for vaporizing said solutions.

Heating devices are currently known which cause the evaporation of vaporizable solutions that contain active insecticidal or deodorant substances. These vaporizable solutions are placed in a container in which an element made of absorbing material, or wick, is inserted; said wick is partially immersed in the solution. The absorbing element is commonly made of high-porosity materials, such as for example paper, felt, and the like, or of low-porosity materials, such as ceramic and the like.

In order to achieve evaporation of the solution, a portion of the absorbing element that protrudes from the container of the vaporizable solution is placed close to the heating means of the device, which is generally powered electrically. A conventional device includes for example a casing that is adapted to be associated with said container and forms the housing for the heating means that are adapted to heat the wick.

The container of the vaporizable solution is generally constituted by a bottle, generally made of transparent plastics or glass. The bottle has a neck from which a portion of the wick, that is partially immersed in the solution, protrudes axially; the neck is threaded externally to screw a container closure thereon.

In said containers there is the problem of avoiding the leakage of the vaporizable solution prior to use, i.e., before the container is associated with the device. The wick immersed in the solution in fact sweats due to capillary action, so that part of the solution can reach the portion that protrudes outside the container and drip from the wick, passing through the region where the closure and the threaded neck of said container are coupled.

This entails several drawbacks, both from the aesthetic point of view, since the solution that drips from the wick visibly soils the outer surface of the container, in addition to sometimes causing the separation of the label applied to said container, and from the functional point of view, since contact with this solution, which is often oily, is unpleasant for the user.

An aim of the present invention is to solve the above problems, providing a container for vaporizable solutions that is capable of ensuring perfect tightness in a closed position.

Another object of the present invention is to provide a container for insecticidal solutions and the like that is simple in concept, safely reliable in operation, and versatile in use.

With this aim and this object in view, there is provided, according to the invention, a container for insecticidal solutions and the like in devices for vaporizing said solutions, of the type that has a neck through which a portion of a wick protrudes, characterized in that it comprises a sealing gasket that is adapted to be fitted by pressing onto said neck and axially forms a tubular passage through which said wick is fitted so that it passes hermetically, said gasket being provided with a lip along its peripheral region, said lip being folded downward and adapted to engage hermetically the upper edge of said neck; and a closure adapted to be screwed on said neck and provided with means adapted to abut against said sealing gasket and axially forming a tubular portion that is closed at the upper end and is adapted to accommodate, with a limited interspace, said portion of the wick that protrudes from said neck of the container.

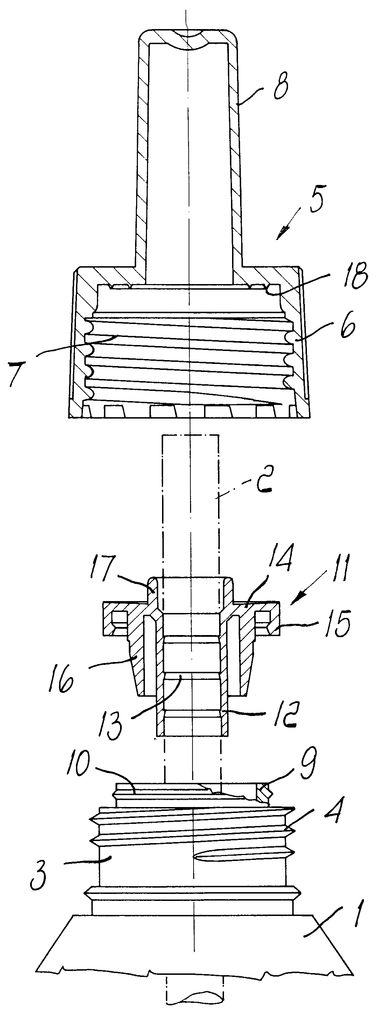

The details of the invention will become apparent from the detailed description of a preferred embodiment of the container for insecticidal solutions and the like, illustrated only by way of non-limitative example in the accompanying drawings, wherein:
the only figure is a partially cutout sectional side view of the container according to the invention.

With particular reference to said figure, the reference numeral 1 designates the container for vaporizable solutions, which is constituted in practice by a bottle made for example of transparent plastic or glass; a portion of an element made of absorbing material, or wick 2, which is partially immersed in the solution protrudes axially from said container 1.

The container 1 has, at the top, a cylindrical neck 3 that is provided, on its outer surface, with a helical protrusion 4 that has a triangular cross-section and is adapted to form the thread for screw thread engagement with a closure 5 of the container.

The closure 5 is formed by a cylindrical body 6 that tapers slightly upwards and is internally provided with a female thread 7 that is adapted to screw onto the thread 4 of the container.

A tubular portion 8 extends axially from the bottom of the body 6 of the closure and is closed at its upper end; in the position for screwing on the closure 5, said tubular portion 8 accommodates, with a limited interspace, the upper portion of the wick 2 that protrudes from the neck 3 of the container.

The neck 3 also has, in an upper portion 9 that has a reduced diameter, an additional circular protrusion 10 that has a triangular cross-section and is adapted to act as retainer for a sealing gasket 11 that is fitted on said neck 3 by pressing.

The gasket 11 forms a tubular passage 12 through which the wick 2 is fitted so that it passes hermetically. Conveniently, the wick 2 has, in a central portion, a series of annular grooves 13 that have a saw-toothed configuration and are adapted for coupling to corresponding ridges that are formed inside the tubular passage 12.

An annular portion 14 protrudes from the top of the tubular passage 12 and is peripherally provided with a lip 15 that is folded downwards and hermetically engages said upper protrusion 10 of the neck 3 of the container. The lip 15 faces a sleeve 16 that protrudes downwards from the annular portion 14 coaxially to the tubular passage 12; said sleeve 16 is adapted to form a seal on the inner surface of the neck 3 of the container.

A ring 17 also protrudes upwards from the annular portion 14; said ring is coaxial to the sleeve 12 and is slightly spaced from the wick 2. Said ring 17 is adapted to engage, in the position for screwing on the closure 5, the inner surface of the tubular portion 8 of said closure 5.

The bottom of the body 6 of the closure 5 also has, on the inner side, a double annular ridge 18 that is adapted to abut, when closed, against the annular portion 14 of the sealing gasket 11.

Therefore, in said closure position the closure 5 is capable of forming a seal on the gasket 11 both frontally, by means of the double circular ridge 18, and peripherally, at the ring 17, which is clamped by the tubular portion 8, and at the lip 15, which surrounds the upper edge of the neck of the container, engaging the retaining protrusion 10.

This ensures the absolute fluid-tightness of the container with respect to the outside, so that the losses of the vaporizable solution observed in conventional containers, with the above-mentioned aesthetic and functional drawbacks, are avoided.

Furthermore, the limited interspace that exists between the tubular portion 8 of the closure 5 and the wick 2 causes the occurrence of very limited sweating due to capillary action. Any liquid that sweats out of the wick 2, dripping along it, does not exit from the closure by virtue of the seal formed by the ring 17 of the gasket 11 on the tubular portion 8, but is instead collected in the space formed by said ring 17. Therefore, when the closure is opened, the neck of the container is also perfectly clean.

An additional advantage offered by the container according to the invention is the fact that the gasket 11 also acts as friction element for the closure, so as to prevent accidental unscrewing of said closure.

In the practical embodiment of the invention, the materials employed, as well as the shape and the dimensions, may be any according to the requirements.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. Container for insecticidal solutions and the like for use in devices for vaporizing said solutions, which comprises a neck through which a portion of an element of absorbing material, or wick, protrudes, characterized in that it comprises a sealing gasket that is adapted to be fitted by pressing onto said neck and axially forms a tubular passage through which said wick is fitted so that it passes hermetically, said gasket being provided with a lip along its peripheral region, said lip being folded downwards and adapted to engage hermetically the upper edge of said neck; and a closure adapted to be screwed on said neck and provided with means adapted to abut against said sealing gasket and axially forming a tubular portion that is closed at the upper end and is adapted to accommodate, with a limited interspace, said portion of the wick that protrudes from said neck of the container.

2. Container according to claim 1, characterized in that said sealing gasket has a front surface from which a ring protrudes in an upward region, said ring being coaxial to said tubular passage and being adapted to hermetically engage the inner surface of said tubular portion of said closure.

3. Container according to claim 2, characterized in that said ring is adapted to be slightly spaced from said wick.

4. Container according to claim 1, characterized in that said closure has, on its bottom, on the inner side, a double annular ridge that is adapted to abut, in closed position, against a front surface of said sealing gasket.

5. Container according to claim 1, characterized in that said neck has, in an upper narrower portion, a circular protrusion that has a triangular cross-section and is adapted to act as a retainer for said lip of said sealing gasket.

6. Container according to claim 1, characterized in that said sealing gasket forms, at the top of said sleeve, an annular portion that is peripherally provided with said lip and from which a sleeve extends downwards, said sleeve being coaxial to said tubular passage and being adapted to form a seal against the inside surface of said neck of the container.
